# EUROPEAN PATENT SPECIFICATION

(11) **EP 0 546 049 B1**
(45) Date of publication and mention of the grant of the patent: **19.07.1995**
(21) Application number: 91915958.2
(22) Date of filing: 28.08.1991
(51) Int. Cl.: C12N 15/67, C12N 15/75, C12N 15/62, C12N 15/31, C12N 15/54, C12N 15/55

(54) **A METHOD FOR RAPID SELECTION OF EFFICIENT SECRETION VECTORS**
EINE METHODE FÜR DIE SCHNELLE SELEKTION VON EFFIZIENTEN SEKRETIONSVEKTOREN
PROCEDE DE SELECTION RAPIDE DE VECTEURS DE SECRETION EFFICACES

(30) Priority: 28.08.1990 US 573759
(43) Date of publication of application: 16.06.1993
(73) Proprietor: E.I. DU PONT DE NEMOURS AND COMPANY, Wilmington Delaware 19898 (US)
(72) Inventor: NAGARAJAN, Vasantha, Wilmington, DE 19807 (US)
(74) Representative: Jones, Alan John
(86) International application number: US9105909
(87) International publication number: WO9203560

(56) References cited:
- EP-A- 0 133 321
- EP-A- 0 288 451
- WO-A-91/00913
- US-A- 4 801 537
- Biochimie, vol. 71, no. 6, June 1989, Elsevier Pub., (Paris, FR) M. Dion et al., pp. 747-755
- Journal of Bacteriology, vol. 171, no. 2, February 1989, American Society for Microbiology (US) C.J.Paddon et al. pp.1185-1187
- Gene, vol. 49, no. 1, 1986, Elsevier (Amsterdam, NL) N. Vasantha et al. pp. 23-28
- Gene, vol. 76, no. 1, 15 March 1989, Elsevier (Amsterdam, NL) N. Vasanthaet al. pp. 53-60

## Description

### FIELD OF THE INVENTION

This invention relates to a method for rapidly selecting a vector which will maximize secretion of a protein by bacteria transformed to produce that protein.

### BACKGROUND OF THE INVENTION

*B. subtilis*, a gram positive bacterium has great potential for producing commercially important proteins because it can be genetically manipulated, adapted to various nutritional and physical conditions of growth, and because it is not pathogenic nor toxogenic to humans. Also, under proper conditions, these bacteria synthesize, translocate and secrete specific proteins relatively free of other proteins, making the proteins easier to purify.

It is generally understood that translocation of secreted proteins across bacterial membranes requires a signal peptide. While a number of studies directed to understand the role of the signal peptide in protein secretion have been done, the mechanism of such translocation and the exact manner the signal peptide influences translocation and removal of the signal peptide from the signal peptide-mature protein complex to yield secreted mature protein is not fully understood.

Vectors enabling the secretion of a number of different heterologous proteins by *B. subtilis* have been demonstrated. See Nagarajan et al., U.S. Patent 4,801,537; Stephens et al., U.S. Patent 4,769,327; and Biotechnology Handbook 2, Bacillus, C. R. Harwood, Ed., Plenum Press, New York (1989). These include vectors that are based on genes for exoenzymes such as amylase, protease, levansucrase and β-lactamases.

Palva et al., demonstrated the secretion by *B*. *subtilis* of the heterologous proteins β-lactamase of *E*. *coli* (*Proc. Natl Acad. Sci. USA* 79, 5582-5586, (1982)) and human leukocyte interferon (*Gene 22*, 229-235, (1983)) by transforming the bacteria with a vector wherein the genes for β-lactamase of *E. coli* and human leukocyte interferon were operably connected to the promoter, ribosome binding site and signal sequence of the α-amylase gene from *Bacillus amyloliquefaciens*. They found that only a low amount of interferon was secreted.

Schein et al., *Biotechnology* 4, 719-725, (1986), performed studies to see why the interferon yields were so low. They, like Palva et al., used essentially the same vector except that the α-amylase signal sequence was accurately fused to the first codon of the mature interferon sequence. The levels of secreted interferon were still only about 1-3% of that of α-amylase. By careful analyses, they concluded that the reduced yields of interferon were not due to the expression of the heterologous interferon gene in *B. subtilis* but were due to the inability of *B. subtilis* to efficiently translocate and/or cleave the interferon-signal peptide complex into the mature interferon.

Ulmanen et al., *J*. *of Becteriol*., Vol. 162, pp. 176-182, (1985) as did Palva et al., and Schein et al., constructed a secretion vector by joining the promoter and signal sequences of the *Bacillus amyloliquefaciens* α-amylase gene in a plasmid, then using the vector they compared the expression and secretion of the heterologous genes coding for TEM-β-lactamase and Semliki Forest virus glycoprotein E1 with that of *Bacillus amyloliquefaciens* α-amylase in *B. subtilis* tranformed with the vectors so created. The yield in the medium (secreted) of TEM-β-lactamase was about 10% and that of E1 was about 0.01% of that of α-amylase. The authors concluded that the low yield of TEM-β-lactamase was due to degradation by host proteases while the low yield of E1 was due to inefficient secretion.

Secretion vectors based on levansucrase were reported by Dion et al., *Biochimie* 71, 747-755, (1989). Dion et al., obtained low levels of mouse interferon compared to levansucrase. The low yield was not due to the secondary structure of the cleavage region. The authors suggested that it was due to a not understood basic incompatability between the signal sequence of the *B. subtilis* levansucrase gene and the mouse interferon α2 gene.

Himeno et al., *FEMS Microbiology Letters* 35, 17-21, (1986) constructed two secretion vector plasmids differing in that one had the signal sequence of *Bacillus licheniformis* penicillinase gene (pen P vector) and the other the signal sequence of *Bacillus stearothermophilus* α-amylase gene (amyT vector). The experimenters then operably linked the DNA sequences encoding 1) penicillin P, 2) amylase T and 3) human salivary α-amylase into each of the vectors and determined the production and secretion into the medium of the three proteins by *B. subtilis* transformed with each of the vectors. They found that the human salivary α-amylase gene was not expressed in either vector; penicillin P was secreted efficiently when incorporated in both vectors; α-amylase was secreted efficiently when incorporated in the amyT vector but only about 3% as efficiently when incorporated in the penP vector. They concluded that not only were a signal sequence and a mature portion of the extracellular enzyme important for protein secretion, but also which combination of the two. There are several differences between the mechanism of secretion of penicillinase and α-amylase and these include signal removal and release into the growth medium. These differences are discussed in the book by Lampen et al., Genetics and Biotechnology of Bacilli, pp. 129-140, Academic Press (1984), herein incorporated by reference. The secretion of penicillinase into the growth medium requires that multiple cleavages and the following set of events occur sequentially: a) signal peptide cleavage, b) modification into a membrane bound intermediate, c) subsequent cleavage, and d) release into the growth medium as shown by Himeno et al., *supra*. In contrast, secretion of α-amylase requires a single signal peptide cleavage and release into the growth medium. Thus the vectors described by Himeno et al. do not enable one to identify the most efficient secretion vector for *B*. *Subtilis* because pencillinase based vectors always have a membrane bound intermediate and are less efficient than amylase based vectors.

It is clear from the various secretion vectors that have been constructed to date that the secretion efficiency of heterologous proteins is different and the reason for the observed difference is not understood. Thus, it is unpredictable what combination of signal peptide and mature protein will result in efficient secretion of the mature protein. Therefore, there is a need for a method to rapidly identify what secretion vector will allow the efficient secretion of any protein one might wish to produce in genetically engineered *B*. *subtilis*. The method of this invention is directed towards providing a solution to this need by enabling the easy combination of DNA sequences encoding promoters, ribosome binding sequences and signal peptides from several sources with the DNA encoding a polypeptide or protein of interest and determining which combination gives the best secretion of mature polypeptide or protein.

### SUMMARY OF THE INVENTION

The present invention comprises a method for selecting a vector which maximizes secretion of a mature protein by bacteria of the genus Bacillus as set forth in Claim 1.

### DETAILED DESCRIPTION OF THE INVENTION

The following definitions are used herein and should be referred to for claim interpretation.
"Precursor protein" is a protein product which includes all of the signal peptide and mature protein
"Mature protein" is the final protein product without any part of the signal peptide attached.
"Desired protein" is any protein considered a valuable product to be obtained from genetically engineered bacteria.
"Secretion efficiency" is the extent to which a precursor protein is converted into a mature protein.
"Signal peptide" is an amino terminal polypeptide preceding the secreted mature protein. The signal peptide is cleaved from and is therefore not present in the mature protein. Signal peptides have the function of directing and translocating secreted proteins across cell membranes. Signal peptide is also referred to as signal protein.
"Compatible restriction sites" are different restriction sites that when cleaved yield nucleotide ends that can be ligated without any additional modification.
"*apr*" is alkaline protease gene.
"*bar*" is extracellular ribonuclease gene.
*"sacB"* or *"lvs"* is levansucrase gene.
*"npr"* is neutral protease gene.
"Shuttle phagemid" is a vector that is double stranded normally and contains both the origins of replication for *E.coli* and *B. subtilis* and also the F1 intragenic region for the prepartion of single stranded DNA.
The terms "peptide", "polypeptide" and "protein" are used interchangeably.
The terms "restriction endonuclease cleavage site" and "restriction site" are used interchangeably.

Suitable methods of genetic engineering employed herein are described in Sambrook et al., Molecular Cloning: A Laboratory Manual - volumes 1,2,3 (Cold Spring Harbor Laboratory: Cold Spring Harbor, New York, 1989), herein incorporated by reference, and in the instructions accompanying commercially available kits for genetic engineering. Bacterial cultures and plasmids necessary to carry out this invention are commercially available and, along with their sources, are identified in the text and examples which follows.

Suitable host bacteria for the vectors of this invention comprise gram positive bacteria belonging to the genus *Bacillus.* These can be obtained from Bacillus Genetics Stock Center (BGSC), The Ohio State University, Columbus, Ohio 43210 or from the American Type Culture Collection, 12301 Parklawn Dr., Rockville, MD 20852. Especially preferred is *B*. *subtilis.*

The method of the present invention includes comparing two or more secretion vectors for their secretion efficiency of any desired protein. Proteins of interest which may be secreted by the method and vector of this invention include but are not limited to:
I. Industrial enzymes from thermophiles and mesophiles
   a) protease
   b) esterase
   c) pectinase
   d) xylanase
   e) amylase
   f) cellulase
   g) levanase
   h) lipase
   i) rnase
   j) nucleotidase
   k) transfructosylase
   l) lactase
   m) glucose isomerase
   n) phosphatase
II. Biotin binding proteins
   avidin
   streptavidin
III. Immunoglobin binding proteins
   a) protein A
   b) protein G
   c) protein L
IV. Immunoglobins
V. Receptor proteins
VI. Structural proteins
   actin
   fibrin
   collagen
   silk proteins
   elastin
VII. Viral proteins
   a) protease
   b) reverse transcriptase
   c) envelope proteins
VIII. Antigens from microbes and protozoa Examples of such proteins include, but are not limited to, staphylococcus protein A, levansucrase, barnase, or streptavidin.

Secretion vectors of this invention include a regulatable promoter sequence which controls transcription, a sequence for a ribosome binding site which controls translation, and a sequence for a signal peptide which enables translocation of the peptide through the bacterial membrane and the cleavage of the signal peptide from the mature protein.

The first component necessary is a vector into which DNA sequences encoding promoter, ribosome binding site and signal peptide from different genes can be engineered. Suitable vectors will be those which are compatible with the bacterium employed. For example, for *B*. *subtilis* such suitable vectors include *E*. *coli-B subtilis* shuttle vectors. They will have compatible regulatory sequences and origins of replication. They will be preferably multicopy and have a selective marker gene, for example, a gene coding for antibiotic resistance. For example, pTZ18R is a phagemid obtainable from Pharmacia, Piscataway, NJ 08854 and confers resistance to ampicillin in *E*. *coli,* and pC194 from BGSC confers resistance to chloramphenicol (cm^{r}) in *E. coli* and *B. subtilis*.

The DNA sequences encoding the promoter, ribosome binding site and signal peptide may be from any single gene which encodes a secreted product. The DNA sequences encoding the promoter and ribosome binding site may also be from a different gene than that encoding the signal peptide. These DNA sequences encoding the promoter, ribosome binding site and signal peptide can be isolated by means well known to those in the art and illustrative examples are documented in the literature. See Biotechnology Handbook 2 Bacillus, C. R. Harwood, Ed., Plenum Press, New York, New York (1989). The promoters in the DNA sequences may be either constitutive or inducible and thus permit the resulting secretion vectors to be differently regulated. Once the best signal peptide sequence is chosen, it is envisioned that promoters from different sources and under different types of control can be independently associated with ribosome binding sites and signal sequences to effect the mode of regulation giving the best yield of a desired protein.

The addition of a restriction endonuclease cleavage site to the 3' end of the DNA encoding the signal peptide is also easily accomplished by means well known to those in the art and is described by Sambrook et al., *supra.* One particularly useful means to add the restriction endonuclease cleavage site to the 3' end of the DNA encoding the signal peptide is by conventional means of site directed mutagenesis as described by Vasantha et al., *Gene*, 76, 53-60, (1989). The means to isolate DNA sequences encoding a desired protein and the addition of restriction sites on the 5' end of the DNA sequence is well known to those in the art and is described by Sambrook et al., *supra.* Any restriction endonuclease site may be used but the use of a restriction site unique to that vector is desirable. The restriction endonuclease site on the 3' end of the DNA sequence encoding the signal peptide and that on the 5' end of the DNA sequence encoding the desired protein must be compatible. Suitable compatible restriction sites are well known in the art. See, for example the Restriction Fragment Compatibility Table of the New England Biolabs 1988-1989 Catalog, New England Biolabs Inc., Beverly, MA 01915 (1988). Preferred for use herein is *EcoRV*. The combined DNA sequences encoding a promoter, ribosome binding site and signal peptide with a restriction site at its 3' end and the DNA sequences encoding mature polypeptides or proteins with a compatible restriction site at its 5' end can be operably integrated by conventional techniques (Sambrook et al., *supra;* Harwood, *supra*).

Once several vectors each with DNA sequences encoding a promoter, ribosome binding site and signal peptide from different genes and compatible restriction sites at the 3' end of the signal peptide coding sequence are available and each is combined with the DNA sequence encoding a desired protein, the vectors are used to transform a suitable bacterium. One conventional method to transform *B. subtilis* bacteria is described by Vasantha et al., *J. of Bacteriol*. 159, 811-819, (1984). Standard microbiological methods well known to those in the art can be used for the growth and maintenance of bacterial cultures.

The efficiency with which the mature protein is secreted into the medium is determined by radiolabeling transformed bacteria containing the vectors and immunoprecipitating the cloned desired polypeptide with antibodies specific for each polypeptide as described by Nagarajan, *Methods in Enzymology* 185, 214-223, (1990), and determining the ratio of the precursor protein and mature protein at different times after the addition of the radioactive material. The immunoprecipated proteins are separated by polyacrylamide gel electrophoresis and visualized by autoradiography. The precursor protein is always bigger in size than the mature protein because it contains all of the signal peptide as well as the mature polypeptide.

Once it is determined which transformed bacteria most efficiently secrete the desired protein, the vector can be recovered by standard methods for recovering plasmids as described by Vasantha et al., *J of Bacteriol*. 159, 811-819, (1984).

From the examples it is clear that secretion efficiency of polypeptides varies depending upon the combination of the signal peptide and mature protein in the vector. By comparing the secretion efficiency of any desired protein which can be easily engineered into vectors containing the DNA encoding different signal peptides with a compatible restriction site on their 3' end, it is possible to choose that vector giving the best secretion of mature desired protein.

The following examples illustrate the method of the present invention but are not intended to limit it in any way. Four secretions vectors were constructed and used in the following examples to demonstrate the method of the invention. The following give the source of the combined DNA sequences encoding the promoter, ribosome binding site and the signal peptide for 1) the alkaline protease gene of *B*. *amyloliquefaciens*, 2) the barnase gene of *B*. *amyloliquefeciens*, 3) the levansucrase gene of *B*. *amyloliquefeciens*, and 4) the neutral protease gene of *B*. *amyloliquefeciens*.

### EXAMPLE 1

### A) CONSTRUCTION OF A VECTOR USING THE DNA SEQUENCE FOR THE PROMOTER, RIBOSOME BINDING SITE AND SIGNAL PEPTIDE FROM THE ALKALINE PROTEASE GENE

The gene for alkaline protease was isolated from *B*. *amyloliquefeciens* by conventional means, for example as described earlier by Vasantha et al; *J*. *Bacteriol* 159, 811-819, (1984). It was inserted into pBE20, an *E*. *coli* - *B*. *subtilis* shuttle phagemid constructed by ligating pTZ18R (Pharmacia, 800 Centennial Ave., Piscataway, NJ 08854) and pC194 (Bacillus Genetic Stock Center (BGSC), Ohio State University, Columbus, Ohio). The resulting vector is pBE25, an *E*. *coli* - *B*. *subtilis* shuttle phagemid vector containing the alkaline protease gene (*apr*) from *B*. *amyloliquefaciens*. An *EcoRV* restriction site was engineered on the 3' end of the DNA sequence encoding the signal peptide of the alkaline protease gene by conventional means of site-directed mutagenesis, for example as described by Vasantha et al, *Gene*, 76, 53-60, (1989), resulting in pBE26.

### B) CONSTRUCTION OF A VECTOR USING THE DNA SEQUENCE FOR THE PROMOTER, RIBOSOME BINDING SITE AND SIGNAL PEPTIDE FROM THE BARNASE GENE

pBE22 is an *E*. *coli* - *B*. *subtilis* shuttle phagemid vector containing the barnase (extracellular ribonuclease) gene from *B*. *amyloliquefaciens*. pBE22 was constructed by subcloning an *EcoR1* fragment from pMT71 (obtained from Dr. Robert Hartley, National Institute of Health, Bethesda, Maryland) (Paddon et al., *J*. *Bacteriol*. 171, 1185-1187 (1989) by standard recombinant DNA techniques (Sambrook et al., *supra*) into pBE20. Preparation of pBE20 is described hereinafter in section C). An *EcoRV* site was engineered a single codon down stream of the barnase signal by site directed mutagenesis (Vasantha & Filpula, supra).

### C) CONSTRUCTION OF A VECTOR USING THE DNA SEQUENCE FOR THE PROMOTER, RIBOSOME BINDING SITE AND SIGNAL PEPTIDE FROM THE LEVANSUCRASE GENE

pBE20 was constructed by ligating *Hind*III digested pTZ18R (Pharmacia, 800 Centennial Ave., Piscataway NJ 08854), which contains an origin of replication for *E*. *coli*, an F1 ori and antibiotic resistance marker ampR, with *Hind*III digested pC194 (Bacillus Stock Center, Ohio State University, Columbus, OH 43210), a *Staphylococcus aureus* plasmid which is a multicopy plasmid in *B*. *subtilis* and which contains a chloramphenicol resistance marker for selection in *B*. *subtilis*.

pBE301 was prepared as follows. The *sacB[BamP]* gene of *B*. *amyloliquefaciens*, which codes for levansucrase (*sacB*), was isolated from a λ ZAP library of the *B*. *amyloliquefaciens* chromosome using oligonucleotide probes as a means of identifying clones containing the appropriate sequences. The *B*. *amyloliquefaciens* λ library was constructed using the commercially available EcoRI-digested λ-ZAP DNA and Giga pack plus (Stratagene, 1109 North Torrey Pines Rd., La Jolla, CA 92037) according to manufacturer's instructions. Two oligonucleotide probes of 30 bases each were synthesized. Probe 52142-2NP had the sequence GACGTTGGGACAGCTGGCCATTACAAAAC and probe 52142-3NP had the sequence ATGAACGGCAAATGGTACCTGTTCACTGAC. A one microliter (400 ng) sample of each probe was 5' end labeled with ³²P λ ATP, as described in Maniatis et al., Molecular Cloning: A Laboratory Manual, p. 122, Cold Spring Harbor Laboratory, Cold Spring Harbor, New York (1982). Equal amounts of the two probes were pooled and mixed. (All methods described in Maniatis et al. are conventional in the art.) *E*. *coli* bacteria, strain BB4, were used as host cells and infected with 5 microliters of the *B*. *amyloliquefaciens* λ ZAP phage. The infected cells were plated to a concentration of 1000 to 1500 plaques/plate on 8 NZYM plates by the pour plate method using 3.0 mL of NZYM top agar. Plates were incubated at 37°C overnight. The DNA from the resulting plaques was transferred to nitrocellulose filters. The filters were then screened for the presence of the *B*. *amyloliquefaciens sac[BamP]* gene using the radio-labeled oligonucleotide probes. The labeled probes (60 ng) were hybridized to the nitrocellulose filters in 6X SSC/0.5% SDS buffer at 37°C for 16 hours. The nitrocellulose filters were washed in 6X SSC/0.1% SDS buffer. The first wash was at room temperature and the second at 37°C. Subsequent autoradiographs of the filters revealed 5 clones which hybridized with the probes. The filters were again washed at 37°C. The resulting autoradiographs of the filters indicated two clones (designated 2A and 2C) potentially containing DNA which hybridized to the probes. Plaques which hybridized with the probes were isolated and phage therein purified according to Maniatis et al., *supra*. The infection and screening procedures were repeated (secondary screen), and about 20% and 80% of the plaques from clones 2A and 2C, respectively, hybridized with the probes. Positive plaques from these secondary screens were then selected, phages prepared, and further screened. Autoradiographs from the tertiary screens showed that all the plaques hybridized the oligonucleotide probes. λ ZAP phage clones 2A and 2C containing the putative *sacB[BamP]* gene sequences were converted into Blue Script plasmid vectors by following the manufacturer's (Stratagene, 1109 North Torrey Pines Rd., La Jolla, CA 92037) instructions. The resulting Blue Script plasmid from λ ZAP 2C clone was designated pBE301. The *sacB[BamP]* fragment from pBE301 was isolated as an *EcoRV-XbaI* fragment and ligated to a *SmaI-XbaI* fragment of pBE20 resulting in pBE501. pBE504 was prepared from pBE501 by adding two new restriction sites in the signal peptide by means of site directed mutation. These added sites did not change the amino acid sequence of the signal peptide.

In pBE504 in order to facilitate heterologous gene fusions an *EcoRV* site was created two codons down stream from the signal peptide processing site by using BioRad Mutagene phagemid in vitro mutagenesis kit (BioRad Laboratories, 1414 Harbor way South, Richmond, CA94804) resulting in pBE311.

### D) CONSTRUCTION OF A VECTOR USING THE DNA SEQUENCE FOR THE PROMOTER, RIBOSOME BINDING SITE AND SIGNAL PEPTIDE FROM THE NEUTRAL PROTEASE GENE

The gene for the neutral protease was isolated from *B*. *amyloliquefaciens* as described by Vasantha et al. *J*. *Bacteriol*. 159, 811-819 (1984) and it resulted in plasmid pBE9. The DNA sequence for the *npr* promoter, ribosome binding site and signal peptide was isolated by polymerase chain reaction (PCR) using the Perkin Elmer thermocycler. Two oligonucleotides
(5'CGGACGATATCCTCAGCGGCCTG3' and
5'ATGCATGGTACCGATCTAACATTTTCCCC3') that annealed to the *npr* gene were used for the PCR reaction and pBE9 was used as the template. The oligonucleotides were designed such that the oligonucleotide that annealed to the 5' end of the promoter encodes a *KpnI* site. The oligonucleotide that annealed to the 3' end of the signal peptide coding region encoded an *EcoRV* site. The PCR product was concentrated and treated with Klenow fragment to fill in any ragged ends followed by digestion with *KpnI* and *EcoRV*. This 340 bp *KpnI-EcoRV* fragment was purified using Geneclean according to manufacturer's instruction (Geneclean Kit, P. O. Box 2284, La Jolla, CA 92038) and provided the *npr* promoter, ribosome binding site and signal peptide for heterologous gene fusions.

### EXAMPLE 2

This Example shows how several different signal peptides can be fused to any given mature heterologous gene using the secretion vectors described in Example 1. The position of the *EcoRV* site with reference to the signal peptide processing site is indicated. The primary amino acid sequence of the signal peptides is also shown. The signal peptides do not share any primary amino acid homology and yet share certain common structural features. The *apr*, *npr*, *bar* and *sacB* signal peptides appear similar and it is not feasible to predict which signal peptide will function better in transporting a heterologous protein.

Staphyloccocal protein A, barnase and levansucrase were used as heterologous proteins. The creation of *EcoRV* site in barnase and levansucrase has been described in Example 1 B and C. An *EcoRV* site in staphyloccocal protein A was created as outlined below.

The source of the staphylococcal protein A gene (*spa*) was a plasmid pRIT5, which was obtained from Pharmacia, 800 Centennial Avenue, Piscataway, NJ 08854. pRIT5 was digested with *Bcl 1* and the 5' overhang was filled in to generate a blunt end compatible with *EcoRV* and later cut with *Pst* and ligated to pBE26 that was digested with *EcoRV* and *Pst*. The ligated DNA was used to transform *B*. *subtilis* and transformants were screened for the production of protein A by colony immunoassay as described by Nagarajan, *supra*. The plasmid DNA was isolated from the colonies producing protein A and had lost both the *EcoRV* site and *Bcl 1* site. An *EcoRV* site was recreated by site directed mutagenesis at the *Bcl 1-EcoRV* junction and the resulting plasmid was designated as pBE45 and has the *apr* promoter and signal peptide fused to staphylococcal protein A (*apr*-spa).

**TABLE 1**

| Hybrid gene⁺ | Plasmid | Source of the Gene | |
|---|---|---|---|
| | | Signal* | Mature |
| Aprₛₛ-Spa | pBE35 to pBE45 | pBE26 | pRIT5 |
| Aprₛₛ-Bar | pBE56 | pBE26 | pBE39 |
| Aprₛₛ-Lvs | pBE335 | pBE45 | pBE311 |
| Nprₛₛ-Spa | pBE80 | PCR product of pBE9 | pBE45 |
| Nprₛₛ-Bar | pBE95 | pBE80 | pBE56 |
| Nprₛₛ-Lvs | pBE83 | pBE80 | pBE342 |
| Barₛₛ-Spa | pBE58 | pBE39 | pBE45 |
| Barₛₛ-Bar | pBE39 | pBE39 | pBE39 |
| Lvsₛₛ-Spa | pBE312 | pBE311 | pBE45 |
| Lvsₛₛ-Bar | pBE336 | pBE311 | pBE56 |
| Lvsₛₛ-Lvs | pBE311 | pBE311 | pBE311 |

| | | | |
|---|---|---|---|
| ⁺The fusions were made at the *EcoRV* site. The signal peptide encoding fragment could be isolated as *Kpn-EcoRV* fragment. | | | |
| *Includes the promoter, ribosome binding site and the signal peptide coding region. | | | |
| The subscript ss refers to signal sequence. | | | |

### EXAMPLE 3

Example 3 illustrates how the secretion efficiency is determined by both the signal peptide and mature protein rather than by either signal peptide or mature sequence alone.

*B*. *subtilis* cells were grown in synthetic medium and labeled with ³H-leucine as described by Borchert and Nagarajan, *J*. *Bacteriol*. 173:276-282 (1991) with the following modification. Strains carrying *Apr*, *Bar* and *Npr* fusion were labelled 30 min after the cell density had reached O.D. 600=0.5. Strains harboring *SacB*-fusions were induced with 2% sucrose when the cell density reached an O.D. 600=0.5 and labelled after 30 min. Bacteria were labeled for 60 sec with ³H-leucine and the radioactive material was chased by the addition unlabeled leucine and samples were withdrawn after 0, 1 and 2 min. An aliquot at 0 time of chase period was transferred to a tube containing 50 uM carbonyl cyanide m-chlorophenyl hydrazine (CCCP) and incubated for 2 min. The samples were processed for immunoprecipitation and separated. Borchert and Nagarajan, *J*. *Bacteriol*. 173:276-282 (1981).

The mobility of the precursor protein (signal peptide + mature protein) and the mature protein is different in a SDS polyacrylamide gel due to the differences in the size. The ratio of the precursor to mature protein at 0 time of chase period is different for the various hybrid proteins.

The rates of signal peptide processing of levansucrase, barnase and protein A were different when fused to Apr signal peptide. Similarly, the rates of signal peptide processing of levansucrase, barnase and protein A were different when fused to Npr signal peptide. The relative secretion efficiency of the various fusions is present in Table 2.

**TABLE 2**

| Signal peptide | Mature protein |
|---|---|
| Aprₛₛ | Lvs > Bar > Spa |
| Nprₛₛ | Bar > Lvs > Spa |
| Barₛₛ | Bar > Spa |
| Lvsₛₛ | Lvs > Spa > Bar |

The relative efficiency of secretion of the mature proteins was determined based on pulse-chase experiments. The rate of signal peptide processing, which is an indication of secretion efficiency was determined by the precursor protein and not by either the signal peptide or mature protein alone.

From the foregoing description, one skilled in the art can easily ascertain the essential characteristics of this invention and, without departing from the scope thereof, can make various changes and modifications to the invention to adapt it to various usages and conditions.

## Claims

1. A method for selecting a genetically engineered vector which maximizes secretion of a mature protein by bacteria of the genus *Bacillus* comprising:
(A) operably connecting in at least two different vectors a first DNA sequence encoding said mature protein to a second DNA sequence encoding a signal protein further operably connected to a third DNA sequence encoding a promoter and a ribosome binding site wherein said DNA sequences encoding said signal proteins in each vector are different from each other, and the 5' end of the DNA sequence encoding each mature protein contains a restriction endonuclease site compatible with the restriction endonuclease site on the 3' end of the DNA sequence encoding said signal protein, said restriction endonuclease site being compatible in each of the different vectors;
(B) transforming bacteria of the genus *Bacillus* with the vectors of (A);
(C) growing said transformed bacteria under conditions suitable for the expression of said vectors whereby a precursor protein comprising a signal protein and a mature protein are expressed and a mature protein is secreted;
(D) determining the ratio of precursor protein to secreted mature protein; and
(E) selecting the vector from the transformed bacteria of the genus *Bacillus* which most efficiently converts precursor protein to mature protein.

2. A method of Claim 1 wherein, in one of said vectors, said DNA sequences encoding both the signal protein and the mature protein are from a single gene.

3. The method of Claim 1 or Claim 2 wherein the bacterium is *B*. *subtilis*.

4. The method of any one of Claims 1 to 3 wherein the promoter and ribosome binding site operably connected to the DNA sequence encoding the signal protein are from the same gene as the signal protein.

5. The method of any one of Claims 1 to 3 wherein the promoter and ribosome binding site operably connected to the DNA sequence encoding the signal protein are from a different gene than the signal protein.

6. The method of any one of Claims 1 to 5 wherein said mature protein comprises staphylococcus protein A.

7. The method of any one of Claims 1 to 5 wherein the mature protein comprises levansucrase.

8. The method of any one of Claims 1 to 5 wherein the mature protein comprises barnase.

9. The method of any one of Claims 1 to 5 wherein the mature protein comprises streptavidin.

10. The method of any one of Claims 1 to 5 wherein the mature protein is selected from the group consisting of industrial enzymes, biotin binding proteins, immunoglobulin binding proteins, immunoglobulins, receptor proteins, viral proteins and antigens of microbial and protozoan origin.

## Patentansprüche

1. Verfahren zum Auswählen eines gentechnisch veränderten Vektors, der die Sekretion eines reifen Proteins durch Bakterien der Gattung *Bacillus* maximiert, umfassend:
(A) in wenigstens zwei verschiedenen Vektoren das operative Verbinden einer das reife Protein kodierenden ersten DNA-Sequenz mit einer ein Signal-Protein kodierenden zweiten DNA-Sequenz, die darüber hinaus mit einer einen Promoter und eine Ribosom-Bindungsstelle kodierenden dritten DNA-Sequenz operativ verbunden ist, wobei die in jedem Vektor die Signal-Proteine kodierenden DNA-Sequenzen voneinander verschieden sind und das jedes reife Protein kodierende 5'-Ende der DNA-Sequenz eine Restriktions-Endonucleasen-Stelle enthält, die mit der Restriktions-Endonucleasen-Stelle am 3'-Ende der das Signal-Protein kodierenden DNA-Sequenz verträglich ist, wobei die Restriktions-Endonucleasen-Stelle in jedem der verschiedenen Vektoren verträglich ist;
(B) das Transformieren von Bakterien der Gattung *Bacillus* mit den Vektoren von (A);
(C) das Züchten der transformierten Bakterien unter Bedingungen, die zur Expression der Vektoren geeignet sind, wobei ein ein Signal-Protein und ein reifes Protein umfassendes Vorläufer-Protein exprimiert werden und ein reifes Protein abgesondert wird;
(D) Das Bestimmen des Verhältnisses von Vorläufer-Protein zu abgesondertem reifem Protein und
(E) Das Selektieren desjenigen Vektors aus den transformierten Bakterien der Gattung *Bacillus*, der am wirkungsvollsten ein Vorläufer-Protein in ein reifes Protein umwandelt.

2. Verfahren nach Anspruch 1, wobei in einem der Vektoren die sowohl das Signal-Protein als auch das reife Protein kodierenden DNA-Sequenzen von einem einzigen Gen stammen.

3. Verfahren nach Anspruch 1 oder Anspruch 2, wobei das Bakterium *B*. *subtilis* ist.

4. Verfahren nach einem der Ansprüche 1 bis 3, wobei die operativ mit der das Signal-Protein kodierenden DNA-Sequenz verbundenen Promotor- und Ribosom-Bindungsstellen aus demselben Gen wie das Signal-Protein stammen.

5. Verfahren nach einem der Ansprüche 1 bis 3, wobei die operativ mit der das Signal-Protein kodierenden DNA-Sequenz verbundenen Promotor- und Ribosom-Bindungsstellen aus einem anderen Gen als dem Signal-Protein stammen.

6. Verfahren nach einem der Ansprüche 1 bis 5, wobei das reife Protein Staphylococcus-Protein A umfaßt.

7. Verfahren nach einem der Ansprüche 1 bis 5, wobei das reife Protein Levansaccharase umfaßt.

8. Verfahren nach einem der Ansprüche 1 bis 5, wobei das reife Protein Barnase umfaßt.

9. Verfahren nach einem der Ansprüche 1 bis 5, wobei das reife Protein Streptavidin umfaßt.

10. Verfahren nach einem der Ansprüche 1 bis 5, wobei das reife Protein aus der aus industriellen Enzymen, biotinbindenden Proteinen, Immunoglobulin-bindenden Proteinen, Immunoglobulinen, Rezeptor-Proteinen, viralen Proteinen und Antigenen mikrobiellen und protozoischen Ursprungs bestehenden Gruppe ausgewählt ist.

## Revendications

1. Un procédé de sélection d'un vecteur modifié par génie génétique qui maximalise la sécrétion d'une protéine mature par des bactéries du genre *Bacillus*, consistant à :
(A) relier fonctionnellement, dans au moins deux vecteurs différents, une première séquence d'ADN codant pour ladite protéine mature à une seconde séquence d'ADN codant pour un peptide signal encore reliée fonctionnellement à une troisième séquence d'ADN codant pour un promoteur et un site de liaison de ribosome, où lesdites séquences d'ADN codant pour lesdits peptides signal dans chaque vecteur sont différentes entre elles, et l'extrémité 5' de la séquence d'ADN codant pour chaque protéine mature contient un site de restriction compatible avec le site de restriction présent sur l'extrémité 3' de la séquence d'ADN codant pour ledit peptide signal, ledit site de restriction étant compatible dans chacun des vecteurs différents ;
(B) transformer des bactéries du genre *Bacillus* avec les vecteurs de (A) ;
(C) faire croître lesdites bactéries transformées dans des conditions convenant à l'expression desdits vecteurs, si bien qu'une protéine précurseur comprenant un peptide signal et une protéine mature est exprimée et qu'une protéine mature est sécrétée ;
(D) déterminer le rapport de la protéine précurseur à la protéine mature sécrétée ; et
(E) sélectionner parmi les bactéries transformées du genre *Bacillus* le vecteur qui convertit le plus efficacement la protéine précurseur en protéine mature.

2. Un procédé de la revendication 1, dans lequel, dans l'un desdits vecteurs, lesdites séquences d'ADN codant pour le peptide signal et pour la protéine mature proviennent toutes deux d'un gène unique.

3. Le procédé de la revendication 1 ou de la revendication 2, dans lequel la bactérie est *B*. *subtilis*.

4. Le procédé de l'une quelconque des revendications 1 à 3, dans lequel le promoteur et le site de liaison de ribosome fonctionnellement reliés à la séquence d'ADN codant pour le peptide signal proviennent du même gène que celui du peptide signal.

5. Le procédé de l'une quelconque des revendications 1 à 3, dans lequel le promoteur et le site de liaison de ribosome reliés fonctionnellement à la séquence d'ADN codant pour le peptide signal proviennent d'un gène différent de celui du peptide signal.

6. Le procédé de l'une quelconque des revendications 1 à 5, dans lequel ladite protéine mature consiste en protéine A de *Staphylococcus*.

7. Le procédé de l'une quelconque des revendications 1 à 5, dans lequel la protéine mature consiste en lévane-sucrase.

8. Le procédé de l'une quelconque des revendications 1 à 5, dans lequel la protéine mature consiste en barnase.

9. Le procédé de l'une quelconque des revendications 1 à 5, dans lequel la protéine mature consiste en streptavidine.

10. Le procédé de l'une quelconque des revendications 1 à 5, dans lequel la protéine mature est choisie dans le groupe formé par les enzymes industrielles, les protéines liant la biotine, les protéines liant les immunoglobulines, les immunoglobulines, les protéines récepteurs, les protéines virales et les antigènes d'origine microbienne et protozoaire.
